# EUROPEAN PATENT SPECIFICATION

(11) **EP 3 052 499 B1**
(45) Date of publication and mention of the grant of the patent: **15.11.2017**
(21) Application number: 14793623.1
(22) Date of filing: 27.09.2014
(51) Int. Cl.: C07D 473/16, A61K 31/52

(54) **CRYSTALLINE ABACAVIR HYDROCHLORIDE MONOHYDRATE AND PROCESS FOR ITS PREPARATION**
KRISTALLINES ABACAVIR-HYDROCHLORID-MONOHYDRAT UND VERFAHREN ZU DESSEN HERSTELLUNG
MONOHYDRATE DE CHLORHYDRATE D'ABACAVIR DE FORME CRISTALLINE ET PROCÉDÉ POUR SA PRÉPARATION

(30) Priority: 03.10.2013 IN 3145MU2013
(43) Date of publication of application: 10.08.2016
(73) Proprietor: Lupin Limited, Mumbai 400 055 (IN)
(72) Inventor: SINGH, Girij, Pal, Pune Maharashtra 412115 (IN); SRIVASTAVA, Dhananjai, Pune Maharashtra 412115 (IN); BHADWAL, Paramvir, Pune Maharashtra 412115 (IN); ANSARI, Inamus, Saqlain, Pune Maharashtra 412115 (IN); PATIL, Sudhakar, Pune Maharashtra 412115 (IN)
(74) Representative: J A Kemp
(86) International application number: PCT/IB2014/064890
(87) International publication number: WO 2015/049623

(56) References cited:
- EP-A2- 2 305 680
- EP-B1- 0 434 450

## Description

### FIELD OF THE INVENTION:

The present invention relates to crystalline abacavir hydrochloride monohydrate and process for its preparation.

### BACKGROUND OF THE INVENTION:

Abacavir (I) is chemically known as (1S, 4R)-4-[2-amino-6-(cyclopropylamino)-9H-purin-9-yl]-2-cyclopentene-1-methanol. Abacavir sulfate and its combination are used in the treatment of human immunodeficiency virus (HIV) infection.

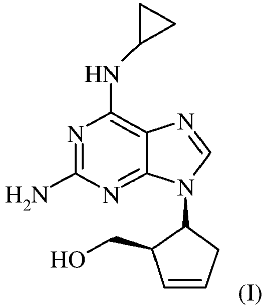

The patent EP 0349242 B1 discloses 9-substituted-2-amino purines including abacavir, process for preparation and pharmaceutical composition thereof.

The patent EP 0434450 B1 describes process for preparation of abacavir hydrochloride by reacting abacavir with 1N hydrochloric acid in ethanol followed by evaporating the solution to dryness. The crude abacavir hydrochloride thus obtained was recrystallized from mixture of ethanol-ethyl acetate to give abacavir hydrochloride 0.8 hydrate.

The physical or chemical properties of a drug can vary depending on the crystalline form of the drug, and such physical and chemical properties can greatly influence a suitable dosage form of the drug, the optimization of a process for preparing the drug, and the *in vivo* absorption of the drug. The discovery of the most appropriate crystalline form of a drug in a procedure for developing the drug enables the development time and cost to be reduced.

### SUMMARY OF THE INVENTION:

The present invention relates to crystalline abacavir hydrochloride monohydrate.

The present invention further provides a process for preparation of crystalline abacavir hydrochloride monohydrate comprising;
a) dissolving abacavir hydrochloride in aqueous C₁-C₄ alcoholic solvent,
b) cooling the solution, and
c) isolating the crystalline solid.

### Brief description of drawings:

Figure 1: X-ray powder differactogram of crystalline abacavir hydrochloride monohydrate.
Figure 2: IR spectrum of crystalline abacavir hydrochloride monohydrate.
Figure 3: TGA of abacavir hydrochloride monohydrate.

### DETAILED DESCRIPTION OF THE INVENTION:

In one embodiment there is provided crystalline abacavir hydrochloride monohydrate.

The characteristic peaks found in XRPD of crystalline abacavir hydrochloride monohydrate are given in the following table.

**Peaks in the XRPD of crystalline abacavir hydrochloride monohydrate:**

| **Position degree 2θ (±0.2)** | **d-spacing** | **I/I₀ (%)** | **Position degree 2θ (±0.2)** | **d-spacing** | **I/I₀ (%)** |
|---|---|---|---|---|---|
| 9.3 | 9.47 | 11.5 | 22.5 | 3.94 | 23.3 |
| 11.9 | 7.42 | 99.8 | 23.1 | 3.83 | 25.1 |
| 13.5 | 6.52 | 15.9 | 23.9 | 3.71 | 19.8 |
| 14.4 | 6.14 | 61.4 | 25.9 | 3.42 | 34.7 |
| 16.4 | 5.37 | 22.8 | 26.2 | 3.39 | 42.5 |
| 18.7 | 4.73 | 59.3 | 27.2 | 3.27 | 13.2 |
| 19.5 | 4.53 | 23.9 | 27.4 | 3.24 | 11.6 |
| 21.7 | 4.08 | 100 | 30.1 | 2.95 | 31.9 |
| 21.9 | 4.03 | 28.7 | 32.4 | 2.75 | 24.2 |

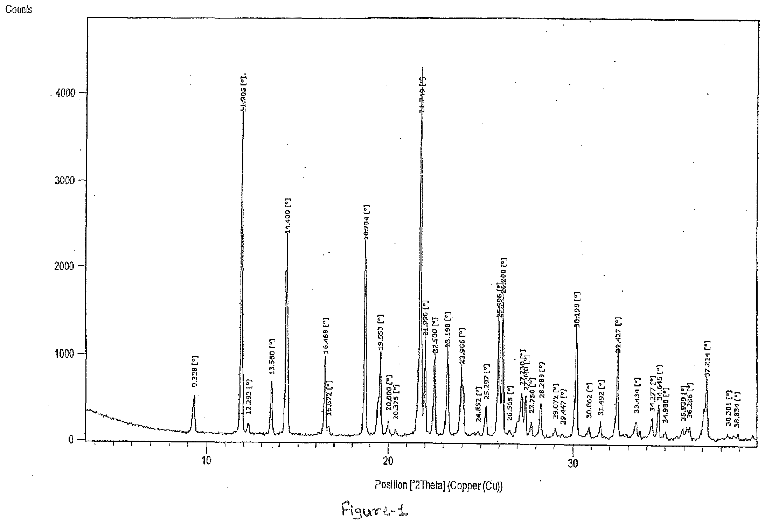

The XRPD of crystalline abacavir hydrochloride monohydrate is as shown in figure 1.

X ray of a single crystal of crystalline abacavir hydrochloride monohydrate shows that the distribution of its atoms in the crystalline network corresponds to the space group P 21 21 21, characterized by the following cell parameters at temperature 298 °K: a= 8.0185 (8) Å; b= 10.9497 (16) Å; c= 18.762 (2) Å; α= β= γ= 90 °.

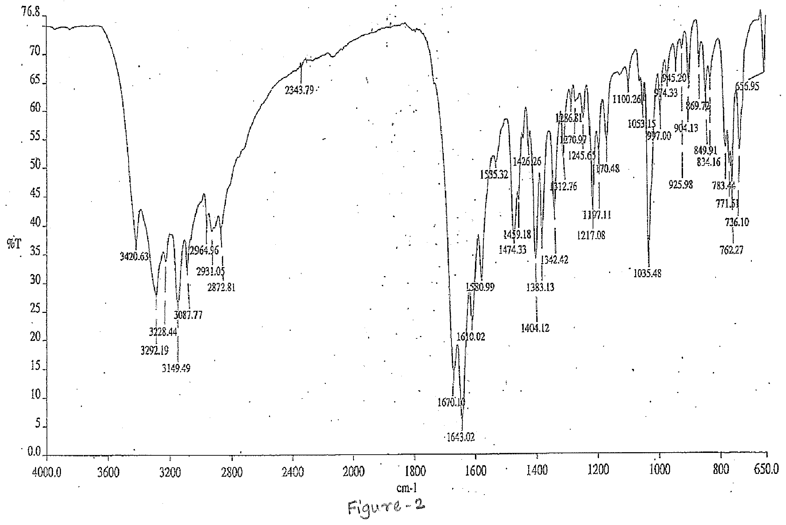

The crystalline abacavir hydrochloride monohydrate is characterized by an infra-red spectrum having major peaks at 3420, 3292, 3087, 1670, 1643 1404, 1217, 10.35 and 762± 5 cm⁻¹. The IR spectrum of crystalline abacavir hydrochloride monohydrate is as shown in figure 2.

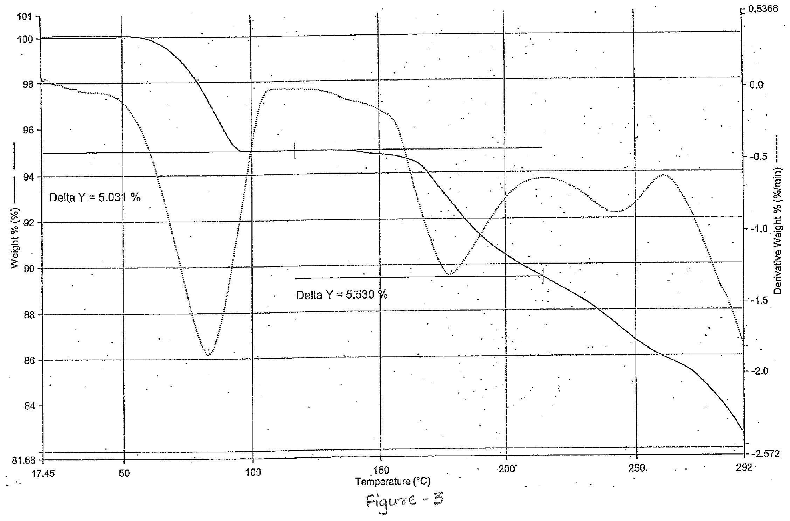

The crystalline abacavir hydrochloride monohydrate characterized by thermogravimetric analysis (TGA) showing Delta Y of 5 % which corresponds to the loss of one water molecule. The TGA of crystalline abacavir hydrochloride monohydrate is shown in figure 3.

The crystalline abacavir hydrochloride monohydrate was found to be stable at 25 ± 2 °C (60 ± 5 RH).

In another embodiment there is provided a process for preparation of crystalline abacavir hydrochloride monohydrate comprising;
a) dissolving abacavir hydrochloride in aqueous C₁-C₄ alcoholic solvent,
b) cooling the solution and
c) isolating the crystalline solid.

Abacavir hydrochloride used for crystallization in the present invention may be prepared as per method known in literature.

The C₁-C₄ alcoholic solvent is selected from methanol, ethanol, n-propanol, isopropanol and butanol, preferably isopropanol.

The aqueous C₁-C₄ alcoholic solvent contains 1 to 20 % of water.

The quantity of aqueous C₁-C₄ alcoholic solvent is 5 to 15 times of abacavir hydrochloride.

The step (a) is carried out by heating which is in the range 40-80 °C, preferably the temperature is between 40-60 °C.

The solution is optionally filtered.

The solution is allowed to cool to ambient temperature, then allowed to cool to -10 to 15 °C, more preferably to 0-5 °C.

Crystalline abacavir hydrochloride can be isolated by methods known in the literature such as filtration, concentration and evaporation etc.

### Experimental:

The powder X-ray diffraction spectrum was recorded at room temperature using PANalytical X'Pert PRO diffractogram with Cu Kα radiation (λ = 1.54060 Å), running at 45 kV and 40 mA.

FTIR spectrum was obtained using a Perkin Elmer Precisely Spectrum 400 instrument using KBr pellet method.

Thermogravimetric analysis (TGA) was done using Pyris-1 TGA Perkin Elmer instrument. The scans were recorded between 30 and 300 °C at a constant heating rate of 10°C/min.

The present invention is described in the following example, however it should be noted that the scope of present invention is not limited by the example.

### Example

### Preparation of Crystalline abacavir hydrochloride monohydrate

Abacavir hydrochloride (70 g) was added in mixture of water (70 ml) and isopropanol (700 ml). The mixture was heated to 50 °C to obtain clear solution. Cooled to 0-5 °C and stirred for 4 hours. The solid was filtered, washed with isopropanol (25 ml) and dried under vacuum. Yield: 50 g, melting point: 126.0-126.8 °C.

## Claims

1. A process for preparation of crystalline abacavir hydrochloride monohydrate comprising;
a) dissolving abacavir hydrochloride in aqueous C₁-C₄ alcoholic solvent,
b) cooling the solution, and
c) isolating the crystalline solid.

2. The process according to claim 1, wherein the C₁-C₄ alcoholic solvent is selected from methanol, ethanol, n-propanol, isopropanol and n-butanol, preferably isopropanol.

3. The process according to claim 1, wherein the aqueous C₁-C₄ alcoholic solvent contains 1 to 20 % of water.

4. The process according to claim 1, wherein the mixture in step (a) is heated to 40 - 80°C.

5. The process according to claim 1, wherein the solvent is 5-15 times of abacavir hydrochloride.

6. The process according to claim 1 wherein the mixture in step (b) is cooled to -10 to 15 °C, preferably 0-5 °C.

7. A crystalline abacavir hydrochloride monohydrate characterized bya powder X-ray diffraction pattern having peaks at
| **Position degree 2θ (±0.2)** | **d-spacing** | **I/I₀ (%)** | **Position degree 2θ (±0.2)** | **d**-**spacing** | **I/I₀ (%)** |
|---|---|---|---|---|---|
| 9.3 | 9.47 | 11.5 | 22.5 | 3.94 | 23.3 |
| 11.9 | 7.42 | 99.8 | 23.1 | 3.83 | 25.1 |
| 13.5 | 6.52 | 15.9 | 23.9 | 3.71 | 19.8 |
| 14.4 | 6.14 | 61.4 | 25.9 | 3.42 | 34.7 |
| 16.4 | 5.37 | 22.8 | 26.2 | 3.39 | 42.5 |
| 18.7 | 4.73 | 59.3 | 27.2 | 3.27 | 13.2 |
| 19.5 | 4.53 | 23.9 | 27.4 | 3.24 | 11.6 |
| 21.7 | 4.08 | 100 | 30.1 | 2.95 | 31.9 |
| 21.9 | 4.03 | 28.7 | 32.4 | 2.75 | 24.2 |

8. A crystalline abacavir hydrochloride monohydrate according to claim 7 **characterized by** a powder X-ray diffraction pattern substantially as depicted in figure 1.

9. A crystalline abacavir hydrochloride monohydrate according to claim 7 **characterized by** an infra-red spectrum substantially as depicted in figure 2.

10. A crystalline abacavir hydrochloride monohydrate according to claim 7 **characterized by** thermogravimetric analysis as shown in figure 3.

## Patentansprüche

1. Verfahren zur Herstellung von kristallinem Abacavir-Hydrochlorid-Monohydrat, umfassend:
a) Auflösen von Abacavir-Hydrochlorid in wässrigem C₁-C₄-alkoholischem Lösungsmittel
b) Abkühlen der Lösung und
c) Isolieren des kristallinen Feststoffes.

2. Verfahren nach Anspruch 1, wobei das C₁-C₄-alkoholische Lösungsmittel aus Methanol, Ethanol, n-Propanol, Isopropanol und n-Butanol, vorzugsweise Isopropanol, ausgewählt ist.

3. Verfahren nach Anspruch 1, wobei das wässrige C₁-C₄-alkoholische Lösungsmittel zu 1 bis 20 % Wasser enthält.

4. Verfahren nach Anspruch 1, wobei das Gemisch in Schritt (a) auf 40-80 °C erhitzt wird.

5. Verfahren nach Anspruch 1, wobei das Lösungsmittel 5-15-fach aus Abacavir-Hydrochlorid ist.

6. Verfahren nach Anspruch 1, wobei das Gemisch in Schritt (b) auf -10 bis 15 °C, vorzugsweise 0-5 °C abgekühlt wird.

7. Kristallines Abacavir-Hydrochlorid-Monohydrat, das durch ein Pulverröntgenbeugungsmuster gekennzeichnet ist, das Spitzen hat bei
| **Position Grad 2θ (±0,2)** | **d-Abstand** | **I/I₀ (%)** | **Position Grad 2θ (±0,2)** | **d-Abstand** | **I/I₀ (%)** |
|---|---|---|---|---|---|
| 9,3 | 9,47 | 11,5 | 22,5 | 3,94 | 23,3 |
| 11,9 | 7,42 | 99,8 | 23,1 | 3,83 | 25,1 |
| 13,5 | 6,52 | 15,9 | 23,9 | 3,71 | 19,8 |
| 14,4 | 6,14 | 61,4 | 25,9 | 3,42 | 34,7 |
| 16,4 | 5,37 | 22,8 | 26,2 | 3,39 | 42,5 |
| 18,7 | 4,73 | 59,3 | 27,2 | 3,27 | 13,2 |
| 19,5 | 4,53 | 23,9 | 27,4 | 3,24 | 11,6 |
| 21,7 | 4,08 | 100 | 30,1 | 2,95 | 31,9 |
| 21,9 | 4,03 | 28,7 | 32,4 | 2,75 | 24,2 |

8. Kristallines Abacavir-Hydrochlorid-Monohydrat nach Anspruch 7, das durch ein Pulverröntgenbeugungsmuster gekennzeichnet ist, das im Wesentlichen wie in Figur 1 dargestellt ist.

9. Kristallines Abacavir-Hydrochlorid-Monohydrat nach Anspruch 7, das durch ein Infrarotspektrum gekennzeichnet ist, das im Wesentlichen wie in Figur 2 dargestellt ist.

10. Kristallines Abacavir-Hydrochlorid-Monohydrat nach Anspruch 7, das durch thermogravimetrische Analyse gekennzeichnet ist, wie in Figur 3 gezeigt.

## Revendications

1. Procédé pour la préparation de chlorhydrate d'abacavir monohydraté cristallin, comprenant :
a) la dissolution de chlorhydrate d'abacavir dans un solvant alcoolique C₁-C₄ aqueux,
b) le refroidissement de la solution, et
c) l'isolement du solide cristallin.

2. Procédé selon la revendication 1, dans lequel le solvant alcoolique C₁-C₄ est sélectionné parmi : méthanol, éthanol, n-propanol, isopropanol et n-butanol, de préférence isopropanol.

3. Procédé selon la revendication 1, dans lequel le solvant alcoolique C₁-C₄ aqueux contient de 1 à 20 % d'eau.

4. Procédé selon la revendication 1, dans lequel le mélange dans l'étape (a) est chauffé jusqu'à 40 à 80°C.

5. Procédé selon la revendication 1, dans lequel le solvant est 5 à 15 fois de chlorhydrate d'abacavir.

6. Procédé selon la revendication 1, dans lequel le mélange dans l'étape (b) est refroidi jusqu'à -10 à 15 °C, de préférence 0 à 5 °C.

7. Chlorhydrate d'abacavir monohydraté cristallin, **caractérisé par** un diagramme de diffraction de rayons X des poudres possédant des crêtes à :
| Degré de position 2θ (±0,2) | espacement d | I/I₀ (%) | Degré de position 2θ (±0,2) | espacement d | I/I₀ (%) |
|---|---|---|---|---|---|
| 9,3 | 9,47 | 11,5 | 22,5 | 3,94 | 23,3 |
| 11,9 | 7,42 | 99,8 | 23,1 | 3,83 | 25,1 |
| 13,5 | 6,52 | 15,9 | 23,9 | 3,71 | 19,8 |
| 14,4 | 6,14 | 61,4 | 25,9 | 3,42 | 34,7 |
| 16,4 | 5,37 | 22,8 | 26,2 | 3,39 | 42,5 |
| 18,7 | 4,73 | 59,3 | 27,2 | 3,27 | 13,2 |
| 19,5 | 4,53 | 23,9 | 27,4 | 3,24 | 11,6 |
| 21,7 | 4,08 | 100 | 30,1 | 2,95 | 31,9 |
| 21,9 | 4,03 | 28,7 | 32,4 | 2,75 | 24,2 |

8. Chlorhydrate d'abacavir monohydraté cristallin selon la revendication 7, **caractérisé par** un diagramme de diffraction de rayons X des poudres sensiblement tel qu'illustré sur la figure 1.

9. Chlorhydrate d'abacavir monohydraté cristallin selon la revendication 7, **caractérisé par** un spectre infrarouge sensiblement tel qu'illustré sur la figure 2.

10. Chlorhydrate d'abacavir monohydraté cristallin selon la revendication 7, **caractérisé par** une analyse thermogravimétrique telle que représentée sur la figure 3.
